# EUROPEAN PATENT APPLICATION

(11) **EP 4 552 669 A1**
(43) Date of publication of application: **14.05.2025**
(21) Application number: 23835577.0
(22) Date of filing: 05.07.2023
(51) Int. Cl.: A61M 5/158, A61M 5/32

(54) **NEEDLE TIP PROTECTOR FOR WINGED NEEDLES**

(30) Priority: 05.07.2022 JP 2022108610
(71) Applicant: Nipro Corporation, Osaka 566-8510 (JP)
(72) Inventor: FUJIWARA, Yuta, Osaka-shi, Osaka 531-8510 (JP)
(74) Representative: Slingsby Partners LLP
(86) International application number: PCT/JP2023/024986
(87) International publication number: WO 2024/010046

(57) **Abstract**

Provided is a needle tip protector (10) for a winged needle that protects the winged needle (46) having two wings (58, 58), wherein: slits (38) into which the wings (58) of the winged needle (46) can be inserted are formed circumferentially between four protective pieces (36, 36, 36, 36), the protective pieces being arranged side by side in a tubular shape; four housing windows (34, 34, 34, 34) connected to the respective slits (38) are provided on a proximal end side of the protective pieces (36); and each slit (38) is connected to the corresponding housing window (34) at a location off a circumferential center and circumferentially opposite ends of the housing window (34).

## Description

### TECHNICAL FIELD

The present invention relates to a needle tip protector for a winged needle that houses and protects a winged needle after use.

### BACKGROUND ART

Conventionally, needle tip protectors for winged needles have been proposed in order to avoid accidental puncture, etc. after removal of winged needles used for dialysis, blood collection, and the like. As shown in, for example, U.S. Patent No. US 6,554,807 B2 (Patent Document 1), a needle tip protector for a winged needle includes protective pieces arranged in a tubular shape. The needle tip of the winged needle is protected by the needle tip protector by being housed in a radial inside of the protective pieces. Such a needle tip protector allows the winged needle to move toward the proximal end side relative to the needle tip protector by the wing of the winged needle being inserted into a slit provided circumferentially between the protective pieces, and allows the needle tip of the winged needle to be housed in the needle tip protector.

### BACKGROUND ART DOCUMENT

### PATENT DOCUMENT

Patent Document 1: US 6,554,807 B2

### SUMMARY OF THE INVENTION

### PROBLEM THE INVENTION ATTEMPTS TO SOLVE

Meanwhile, when a winged needle has two wings that extend toward the opposite outer sides in the axis-perpendicular direction, it is also conceivable that four protective pieces are provided so as to form slits every 90 degrees in the circumferential direction, thereby making it easier to insert the wings into the slits. In such a structure, it is planned that the two wings are inserted into the slits located on opposite sides in the diametrical direction, and that the two protective pieces are located in one circumferential interval between the two wings, and the other two protective pieces are located in the other circumferential interval between the two wings.

However, it has been discovered that, depending on the orientation of the winged needle or inclination of the wing in the circumferential direction during insertion of the wing into the slit, for example, two wings may be inserted into the slits provided on circumferentially opposite sides adjacent to one protective piece, resulting in one protective piece being located in one circumferential interval between the two wings and three protective pieces being located in the other circumferential interval, which is different from what was expected. In this case, some kind of impediment to the operation or function may be caused, for example, the protective piece may be deformed to adversely affect the function of protecting the winged needle, or the like.

It is therefore one object of the present invention to provide a needle tip protector for a winged needle of novel structure which is able to solve at least one of the above-mentioned problems that can occur when four protective pieces are provided.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; and four housing windows each provided on a proximal end side of the corresponding protective piece, each housing window being connected to the corresponding slit, wherein each slit is connected to the corresponding housing window at a location off a circumferential center and circumferentially opposite ends of the housing window.

In the case where the two wings of the winged needle are inserted into the respective slits provided on the opposite sides of a single protective piece, each wing is housed in the corresponding housing window while inclining in the circumferential direction. Thus, the wing within the housing window is easily pressed against the wall inner surface of the housing window in the circumferential direction. In this case, according to the needle tip protector structured following the present preferred embodiment, the connected portion of the slit connected to the housing window is set at the location off the circumferentially opposite ends of the housing window. Thus, the wing located at the end of the housing window in the circumferential direction finds it difficult to return to the slit. Therefore, by preventing the winged needle from moving toward the needle tip side relative to the needle tip protector, re-exposure of the needle tip of the winged needle to the distal end side can be prevented, thereby preventing occurrence of accidental puncture due to the re-exposure of the needle tip.

Besides, the connected location of the slit connected to the housing window is set at the location off the circumferential center of the housing window. Thus, for example, by locating the wing housed in the housing window at the circumferential center within the housing window, it is possible to prevent the wing from returning into the slit.

A second preferred embodiment provides the needle tip protector according to the first preferred embodiment, wherein a sloping part sloping in a circumferential direction is provided in a middle of each slit, and each slit is configured such that opposite sides of the sloping part are shifted from each other in the circumferential direction.

According to the needle tip protector structured following the present preferred embodiment, by providing the sloping part in the middle of each slit, even if the four protective pieces have approximately the same shape and the opening location of each slit on the distal end side is set at the circumferential center of the corresponding housing window, the connected location of the slit connected to the housing window can be set at the location off the circumferential center of the housing window. With this configuration, for example, in the case where two protective pieces are located in one circumferential interval between the two wings, and the other two protective pieces are located in the other circumferential interval between the two wings, even if the winged needle, which has entered the housing window from the slit, moves to the circumferential center due to the reaction force of torsion of the tube, which is connected to the winged needle, during passing through the sloping part or the like, the wing housed in the housing window will find it difficult to return to the slit, thereby preventing re-exposure of the winged needle to the distal end side.

A third preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; four housing windows each provided on a proximal end side of the corresponding protective piece, each housing window being connected to the corresponding slit; and columnar parts separating the housing windows that are adjacent to each other in a circumferential direction, each columnar part being provided circumferentially between adjacent ones of the housing windows while extending from the corresponding protective piece to the proximal end side, wherein a circumferential angle of each columnar part about a center axis is not greater than 30 degrees.

According to the needle tip protector structured following the present preferred embodiment, for example, in the case where the two wings of the winged needle are inserted into the respective slits on circumferentially opposite sides of a single protective piece, the force exerted on the columnar part and the protective piece by the wings being pressed against the columnar part can be reduced. Therefore, with the winged needle housed and protected by the needle tip protector, displacement of the protective piece due to contact by the wing can be suppressed, thereby preventing exposure of the needle tip due to the mutual separation of the protective pieces in the circumferential direction and in the radial direction.

A fourth preferred embodiment provides the needle tip protector according to the third preferred embodiment, wherein the circumferential angle of each columnar part is not smaller than 10 degrees.

According to the needle tip protector structured following the present preferred embodiment, it is possible to reduce the force exerted by the wings being pressed against the columnar part, while reliably obtaining the strength of the columnar part. This makes it possible to effectively exhibit the needle tip protective function by suppressing the displacement of the protective piece due to deformation of the columnar part.

A fifth preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; four housing windows each provided on a proximal end side of the corresponding protective piece, each housing window being connected to the corresponding slit; a tube insertion part provided on the proximal end side of the housing windows, the tube insertion part being configured to allow a tube connected to the proximal end side of the winged needle to be inserted; and an annular retaining part provided at an end of the tube insertion part on a side of the housing windows, the annular retaining part being continuous about an entire circumference, wherein an inner circumferential surface of the annular retaining part has a quadrangular cross-sectional shape including corners at four locations in a circumferential direction.

In the case where the two wings of the winged needle are inserted into the respective slits provided on the opposite sides of a single protective piece, the tube extending to the proximal end side of the winged needle may be pressed against the end of the tube insertion part on the side of the housing windows. In this case, according to the needle tip protector structured following the present preferred embodiment, the inner circumferential surface of the annular retaining part, against which the tube is pressed, has a quadrangular cross-sectional shape. Thus, the tube is easily guided toward the corners of the annular retaining part, and the location on which the external force acts due to contact by the tube can be set to the corners of the annular retaining part. Therefore, the external force due to contact by the tube will be prevented from acting on the needle tip protector at unintended locations in the circumferential direction, thereby stably obtaining the needle tip protective function or the like.

A sixth preferred embodiment provides the needle tip protector according to the fifth preferred embodiment, wherein the tube insertion part has a divided structure divided in the circumferential direction such that the four protective pieces are separated into two pieces each, and the corners of the inner circumferential surface of the annular retaining part are shifted from a divided location of the tube insertion part in the circumferential direction.

According to the needle tip protector structured following the present preferred embodiment, by preventing the external force due to contact by the tube from acting on the divided location of the tube insertion part, it is possible to prevent troubles such as the divided location of the tube insertion part accidentally opening due to the action of the external force.

A seventh preferred embodiment provides the needle tip protector according to the fifth or sixth preferred embodiment, wherein a tube retaining surface configured to retain the tube is provided on an inner surface of the tube insertion part, a tube retaining groove opening toward the tube is formed at an end of the tube retaining surface on the side of the housing windows, and the tube retaining groove is located on the proximal end side of each corner of the annular retaining part in the circumferential direction.

According to the needle tip protector structured following the present preferred embodiment, by positioning and holding the tube in the tube retaining groove, it is possible to prevent the tube from being easily guided to a location other than the corner of the annular retaining part due to contact with the tube retaining surface. Therefore, the tube will be stably held in the circumferential location where the corner of the annular retaining part is provided.

An eighth preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; four housing windows each provided on a proximal end side of the corresponding protective piece, each housing window being connected to the corresponding slit; and a tube insertion part provided on the proximal end side of the housing windows, the tube insertion part being configured to allow a tube connected to the proximal end side of the winged needle to be inserted, wherein a tube retaining surface configured to retain the tube is provided on an inner surface of the tube insertion part, and a tube retaining groove opening toward the tube is formed at an end of the tube retaining surface on a side of the housing windows.

In the case where the two wings of the winged needle are inserted into the respective slits provided on the opposite sides of a single protective piece, the tube connected to the winged needle may be pressed against the tube retaining surface of the tube insertion part and move along the tube retaining surface. In this case, according to the needle tip protector structured following the present preferred embodiment, the tube is positioned and held by the tube retaining groove opening onto the tube retaining surface. This makes it possible to control the location where the tube is pressed against the inner surface of the tube insertion part. Therefore, it is possible to prevent the external force acting on the tube insertion part from adversely affecting the needle tip protective function or the like of the needle tip protector.

A ninth preferred embodiment provides a needle tip protector for a winged needle configured to protect the winged needle having two wings, comprising: four protective pieces arranged side by side in a tubular shape; and slits configured to allow the wings of the winged needle to be inserted, each slit being formed circumferentially between adjacent ones of the protective pieces, wherein a sloping part sloping in a circumferential direction is provided in a middle of each slit, and each slit is widened at the sloping part.

According to the needle tip protector structured following the present preferred embodiment, by providing the sloping part in the middle of the slit, the path of the slit can be set with a large degree of freedom. In addition, since the slit is widened at the sloping part, when the wing of the winged needle passes through the sloping part, sliding resistance can be reduced and the wing can be prevented from being caught, and the like, thereby allowing the wing to move smoothly within the slit.

A tenth preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; and four housing windows each provided on a proximal end side of the corresponding protective piece, each housing window being connected to the corresponding slit while being wider than the corresponding slit in a circumferential direction , wherein each slit includes a widened part whose width dimension is partially increased, the widened part being provided at a location away from the corresponding housing window to a distal end side.

According to the needle tip protector structured following the present preferred embodiment, for example, by shortening the length of the constricted portion of the slit provided between the housing window and the widened part of the slit, when the wing passes through the said constricted portion, the end of the wing will be located in the housing window or the widened part, and inclination of the wing in the axial direction is allowed. This reduces the force acting on the protective piece from the wing, thereby suppressing the action in which the protective piece is pushed open by the wing.

An eleventh preferred embodiment provides a needle tip protector for a winged needle configured to protect the winged needle having two wings, comprising: four protective pieces arranged side by side in a tubular shape; slits configured to allow the wings of the winged needle to be inserted, each slit being formed circumferentially between adjacent ones of the protective pieces; and four housing windows each provided on a proximal end side of the corresponding protective piece, each housing window being connected to the corresponding slit, wherein in every state where the two wings are inserted into any two of the housing windows that are adjacent to each other in a circumferential direction, an axial length of each housing window is larger than that of an inserted portion of each wing inserted into the corresponding housing window such that the inserted portion of each wing is housed within the corresponding housing window.

When the wing passes through the slit and enters the housing window, normally, the linking part of the wing would pass through the slit and enter the housing window. However, for example, if the wing passes through the slit while undergoing strain deformation and enters the housing window, the two wings may be inserted into the respective two slits located on the circumferentially opposite sides interposing a single protective piece, resulting in a 3:1 insertion state as described later. In such a 3:1 insertion state, the outer part of each wing, which has an axial length longer than that of the part closer to the needle hub, may enter the housing window through the slit. Thus, according to the needle tip protector structured following the present preferred embodiment, the axial length dimension of the housing window is made larger than that of the inserted portion of each wing inserted into the housing window in the said 3:1 insertion state. Therefore, even in the case where the two wings pass through the slits that are adjacent to each other in the circumferential direction and result in the 3:1 insertion state as described later, the wings are stably and reliably housed in the housing windows, and the return of the wings from the housing windows to the slits is effectively suppressed, thereby stably exhibiting prevention of re-exposure of the winged needle to the distal end side.

### EFFECT OF THE INVENTION

According to the present invention, it is possible to prevent adverse effects or the like on the function or operation of protecting the needle tip that may occur in a needle tip protector for a winged needle including four protective pieces.

### BRIEF DESCRIPTION OF THE DRAWINGS

FIG. 1 is a perspective view of a needle tip protector for a winged needle according to a first practical embodiment of the present invention.
FIG. 2 is a front view of the needle tip protector shown in FIG. 1.
FIG. 3 is a top plan view of the needle tip protector shown in FIG. 1.
FIG. 4 is a bottom plan view of the needle tip protector shown in FIG. 1.
FIG. 5 is an enlarged cross-sectional view taken along line 5-5 of FIG. 2.
FIG. 6 is an enlarged cross-sectional view taken along line 6-6 of FIG. 2.
FIG. 7 is an enlarged cross-sectional view taken along line 7-7 of FIG. 2.
FIG. 8 is a perspective view of the needle tip protector shown in FIG. 1 with its divided portion opened.
FIG. 9 is a front view of the needle tip protector shown in FIG. 8.
FIG. 10 is a front view of the needle tip protector shown in FIG. 1 attached to the winged needle.
FIG. 11 is a left side view of the needle tip protector shown in FIG. 10 attached to the winged needle.
FIG. 12 is a cross-sectional view taken along line 12-12 of FIG. 11.
FIG. 13 is a front view of a situation where the winged needle is housed in the needle tip protector shown in FIG. 1 in a normal state.
FIG. 14 is a left side view of the situation where the winged needle is housed in the needle tip protector shown in FIG. 13 in the normal state.
FIG. 15 is a cross-sectional view taken along line 15-15 of FIG. 14.
FIG. 16 is a top plan view of the situation where the winged needle is housed in the needle tip protector shown in FIG. 13 in the normal state.
FIG. 17 is a view of a situation where the winged needle is housed in the needle tip protector shown in FIG. 1 with wings unevenly inserted, corresponding to a view as seen in a direction indicated by arrow 17 in FIG. 18.
FIG. 18 is a top plan view of the situation where the winged needle is housed in the needle tip protector shown in FIG. 13 with the wings unevenly inserted.
FIG. 19 is an enlarged cross-sectional view taken along line 19-19 of FIG. 17.
FIG. 20 is a view showing a process in which the winged needle is housed in the needle tip protector shown in FIG. 1 with the wings unevenly inserted.

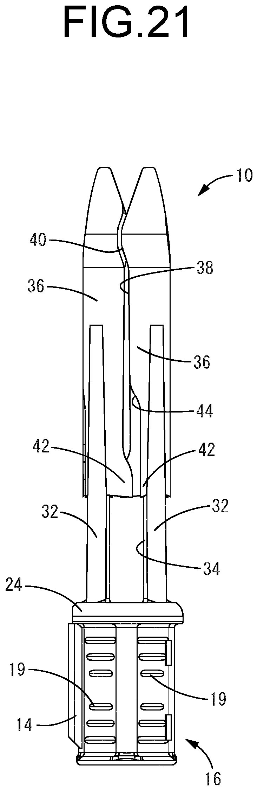

FIG. 21 is a rear view of the needle tip protector shown in FIG. 2 according to the first practical embodiment.

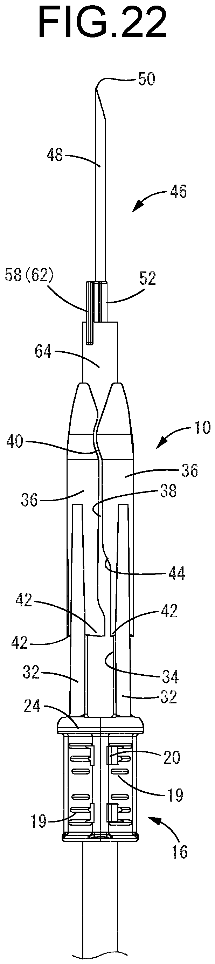

FIG. 22 is a rear view of the needle tip protector shown in FIG. 11 according to the first practical embodiment.

### EMBODIMENTS FOR CARRYING OUT THE INVENTION

Hereinafter, a practical embodiment of the present invention will be described with reference to the drawings.

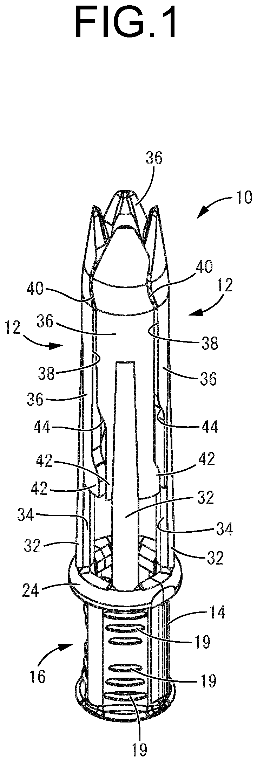

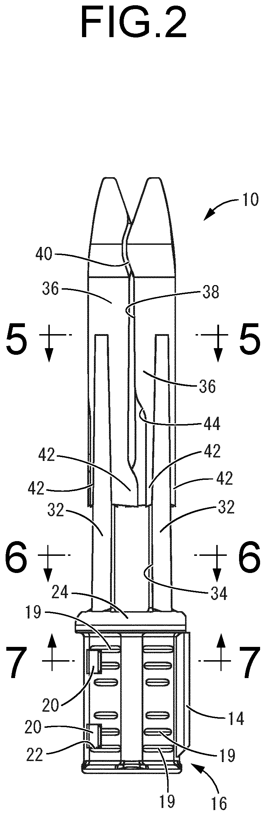

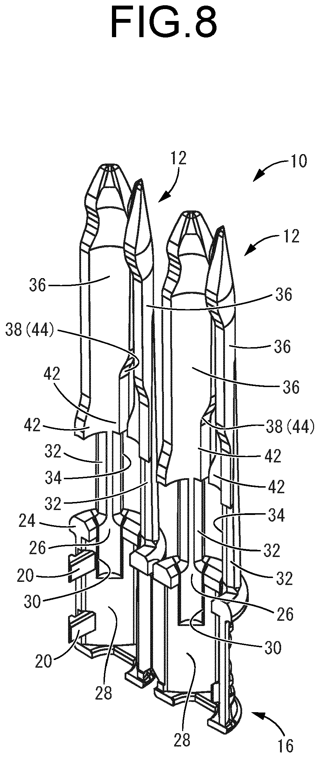

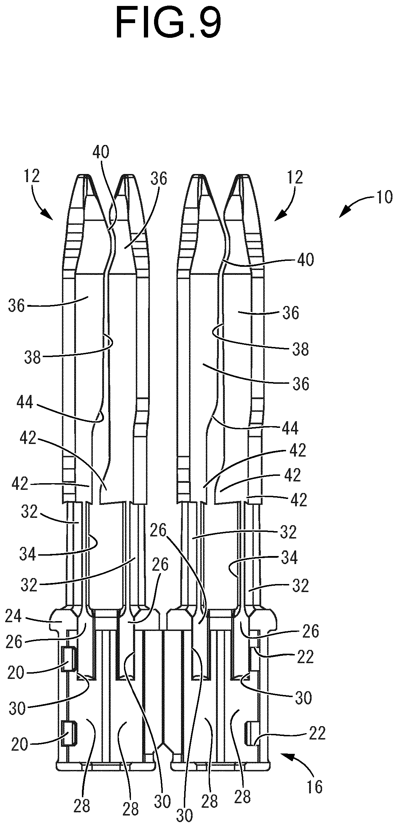

FIGS. 1 to 7 show a needle tip protector 10 for a winged needle (hereinafter referred to as the "needle tip protector 10") according to a first practical embodiment of the present invention. The needle tip protector 10 has a tubular shape overall. As shown in FIGS. 8 and 9, the needle tip protector 10 has a divided structure comprising two division units 12, 12 that are divided by half a circumference, and these division units 12, 12 are integrally coupled by a hinge 14 at the ends on one circumferential side. In the following description, as a general rule, the distal end side refers to the upper side in FIG. 2, which is a needle tip 50 side of a winged needle 46 described later, and the proximal end side refers to the lower side in FIG. 2, which is a tube 64 side of the winged needle 46 described later. Besides, as a general rule, the axial direction refers to the center axis direction of the tubular needle tip protector 10, and the circumferential direction refers to the circumferential direction of the needle tip protector 10.

The hinge 14 coupling the division units 12, 12 is provided in a tube insertion part 16, which constitutes the proximal end part of the needle tip protector 10. The tube insertion part 16 has a tubular shape that can be externally fitted onto the tube 64 described later. A proximal end opening 18 of the tube insertion part 16 has an approximately elliptical shape, and the diameter dimension in the minor axis direction is approximately the same as the outer diameter dimension of the tube 64 described later. A plurality of anti-slip projections 19 are formed on the outer circumferential surface of the tube insertion part 16 and extend in the circumferential direction. With this configuration, when, for example, the tube insertion part 16 is picked up with the fingertips and an axial force is applied, the fingertips are less likely to slip and the force is readily applied.

In addition, the tube insertion part 16 includes a fixing means that holds the tube insertion part 16 in a tubular shape at the portion that is opposed to the hinge 14 in the diametrical direction. The circumferential ends of the division units 12, 12 opposite the hinge 14 are overlapped with each other in the circumferential direction to be coupled and fixed by the fixing means, thereby holding the tube insertion part 16 in a tubular shape. In this way, the tube insertion part 16 has a divided structure having a circumferential divided portion, and the ends on one circumferential side are integrally continuous with each other by the hinge 14, while the ends on the other circumferential side are coupled and fixed by the fixing means. The fixing means of the present practical embodiment is constituted by coupling hooks 20, which protrude outside in the circumferential direction from one division unit 12 and each have a claw at its distal end part, being inserted through respective coupling holes 22, which are provided in the other division unit 12, to be locked to the opening peripheral edges of the coupling holes 22.

The distal end part of the tube insertion part 16 constitutes an annular retaining part 24. The annular retaining part 24 is provided continuously about the entire circumference. The outer circumferential surface of the annular retaining part 24 has a transverse cross section (an axis-perpendicular cross section) of approximately circular shape. Meanwhile, inner circumferential surface of the annular retaining part 24 has a transverse cross section of approximately quadrangular shape including four corners 26, 26, 26, 26.

Regarding the tube insertion part 16, the inner circumferential surface on the proximal end side of the annular retaining part 24 is constituted by four tube retaining surfaces 28, 28, 28, 28. Each tube retaining surface 28 is an approximately flat surface. The inner circumferential surface of the tube insertion part 16, which is constituted by the four tube retaining surfaces 28, 28, 28, 28, has an approximately quadrangular cross-section.

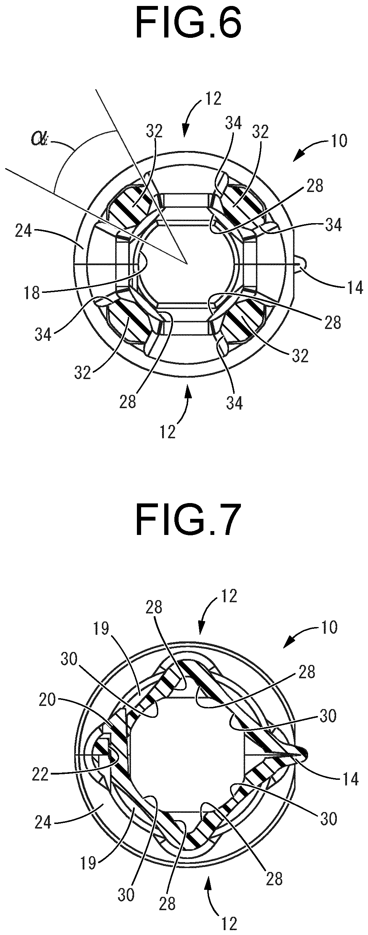

As shown in FIGS. 7 to 9, a tube retaining groove 30 is formed in each tube retaining surface 28 of the tube insertion part 16. The tube retaining groove 30 is a groove-shaped recess that opens on the tube retaining surface 28 and extends in the axial direction, and is provided at the end of the tube retaining surface 28 on the distal end side. In the present practical embodiment, the groove inner surface of the tube retaining groove 30 has a curved surface with an approximately arcuate cross section. However, the shape of the groove inner surface of the tube retaining groove 30 is not particularly limited, and may be, for example, a surface with a polygonal cross section constituted by a combination of multiple planes, or the like.

Regarding the inner circumferential surface of the tube insertion part 16, the portion constituted by the annular retaining part 24 and the portion constituted by the four tube retaining surfaces 28, 28, 28, 28 are oriented such that they are shifted from each other by 90 degrees in the circumferential direction. Each corner 26 of the inner circumferential surface of the annular retaining part 24 is arranged so as to be located side by side with the circumferential center of the corresponding tube retaining surface 28 in the axial direction. The tube retaining groove 30 is provided on the proximal end side of each corner 26 of the inner circumferential surface of the annular retaining part 24, and the groove inner surface of the tube retaining groove 30 is smoothly continuous with the inner circumferential surface of the annular retaining part 24 without any step.

The corners 26, 26, 26, 26 of the annular retaining part 24 and the tube retaining grooves 30, 30, 30, 30 are all shifted in the circumferential direction from the divided location, which comprises the circumferential ends of the division unit 12, 12. In the present practical embodiment, the circumferential ends of the division units 12, 12 (the divided location of the tube insertion part 16) are located approximately in the center between the corners 26, 26 on the inner circumferential surface of the annular retaining part 24, which has an approximately quadrangular transverse cross section. With this arrangement, the distance between the corners 26 and the divided location of the tube insertion part 16 is made large.

As shown in FIGS. 1 and 6, there are provided four columnar parts 32, 32, 32, 32 extending from the annular retaining part 24 toward the distal end side at the portions corresponding to the corners 26, 26, 26, 26 of the inner circumferential surface of the annular retaining part 24 in the circumferential direction. Each columnar part 32 has a rod shape extending in a straight line with approximately a constant cross-sectional shape. The columnar part 32 of the present practical embodiment has an approximately octagonal cross-sectional shape, but the cross-sectional shape of the columnar parts 32 is not particularly limited, and may be circular or the like, for example. The four columnar parts 32, 32, 32, 32 are arranged approximately at regular intervals in the circumferential direction, and two columnar parts 32, 32 are provided in each division unit 12. The circumferential angle α of the columnar part 32 about the center axis of the needle tip protector 10 shown in FIG. 6 is preferably not greater than 30 degrees, and more preferably not greater than 25 degrees. Besides, the circumferential angle α of the columnar part 32 is preferably not smaller than 10 degrees, and more preferably not smaller than 15 degrees. The circumferential angle α of the columnar part 32 about the center axis of the needle tip protector 10 is the angle formed by the tangents at the circumferentially opposite ends of the columnar part 32 passing through the center of the needle tip protector 10. Here, the tangent refers to a straight line passing through a single point on the outer circumferential surface of the columnar part 32, and not only when the transverse cross section of the columnar part 32 has an outer shape constituted by a curve such as a circle and an ellipse, but also when the transverse cross section of the columnar part 32 has a polygonal shape, for example.

A housing window 34 is formed circumferentially between the columnar parts 32, 32 arranged adjacent to each other in the circumferential direction. As shown in FIGS. 2 and 9, the housing window 34 is provided with an approximately rectangular aperture shape penetrating in the radial direction, and is surrounded by the columnar parts 32, 32 on the circumferentially opposite sides, the annular retaining part 24 on the proximal end side, and a protective piece 36 (described later) on the distal end side. The needle tip protector 10 includes four housing windows 34, 34, 34, 34 which have approximately the same shape and size. The housing windows 34, 34, 34, 34 are arranged approximately at regular intervals in the circumferential direction, and the housing windows 34, 34 that are adjacent to each other in the circumferential direction are separated by the columnar part 32. The wall on the proximal end side of the housing window 34 is constituted by the annular retaining part 24, and the tube insertion part 16 is located on the proximal end side of the housing windows 34.

As shown in FIGS. 1, 2, 8, and 9, a protective piece 36 is provided on the distal end side of each columnar part 32. The protective piece 36 has a plate shape that is curved in an arcuate shape in the circumferential direction, and has a width dimension of about a quarter of the circumference in the circumferential direction. The protective piece 36 has a tapered shape whose distal end portion becomes narrower in the circumferential direction toward the distal end. The distal end portion of the protective piece 36 is curved so that one circumferential end face is convex and the other circumferential end face is concave. In the protective piece 36, the portion on the distal end side of a sloping part 44 of a slit 38 (described later) is configured such that its circumferential center is located on the extension of the columnar part 32 toward the distal end side. The distal end portion of the protective piece 36 has a curved shape corresponding to a curved part 40 of the slit 38, but as a whole, its circumferential center is located on the extension of the columnar part 32. In particular, the protective piece 36 is configured such that its circumferential center is located on the extension of the columnar part 32 in a part between the sloping part 44 and the curved part 40, and in a part on the distal end side of the curved part 40.

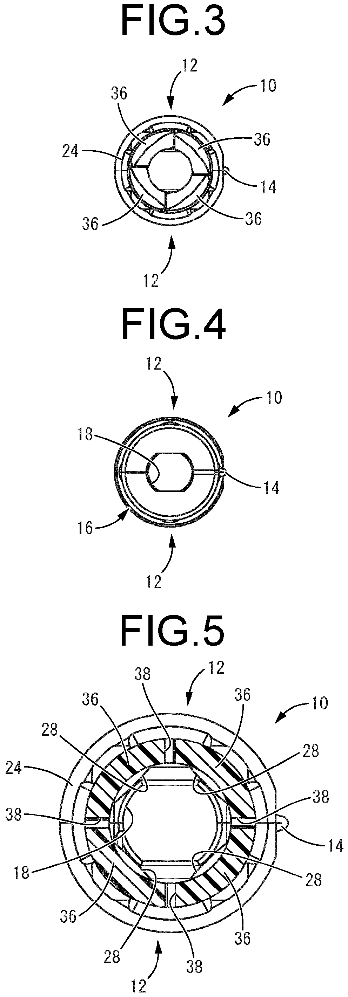

Two protective pieces 36 are provided on each of the two division units 12, 12, and in the entire needle tip protector 10, four protective pieces 36, 36, 36, 36 are arranged side by side in the circumferential direction. The four protective pieces 36, 36, 36, 36 form a tubular shape. Each columnar part 32 extends from the corresponding protective piece 36 towards the proximal end side, and the protective piece 36 and the tube insertion part 16 are coupled by the columnar part 32. In the present practical embodiment, the tube insertion part 16, the columnar parts 32, and protective pieces 36 are integrally formed using synthetic resin or the like, but they may be formed as separate members and fixed to each other. The columnar part 32, the cross-sectional shape of which is shown in FIG. 6, is located approximately in the center in the circumferential direction of the middle portion of the protective piece 36, which is located axially between the curved distal end portion whose cross-sectional shape is shown in FIG. 5 and the sloping part 44 of the slit 38 (described later). As shown in FIGS. 5 and 6, the said middle portion of the protective piece 36 and the columnar part 32 are both arranged approximately at regular intervals in the circumferential direction.

A slit 38 is formed circumferentially between the protective pieces 36, 36 that are adjacent to each other in the circumferential direction. The slit 38 extends continuously along the entire length of the protective piece 36 from the distal end to the proximal end. The distal end of the slit 38 opens toward the distal end side between the protective pieces 36, 36. In the present practical embodiment, the distal end portion of the protective piece 36 becomes narrower in the circumferential direction toward the distal end, and the distal end side of the slit 38 becomes expanded in the circumferential direction toward the distal end. In addition, because a part of the distal end portion of the protective piece 36 has a curved shape, the distal end portion of the slit 38 constitutes a curved part 40 that extends in a curved shape.

The proximal end of the slit 38 is connected to the housing window 34. The housing window 34 is wider than the proximal end of the slit 38. In the present practical embodiment, the housing window 34 is wider than the sloping part 44 (described later) that is partially widened in the slit 38. The proximal end of the slit 38 is located off the circumferentially opposite ends of the housing window 34 in the circumferential direction. The circumferentially opposite end parts of the protective piece 36 protrude from the columnar part 32 to the opposite outer sides in the circumferential direction and constitute detent parts 42. The slit 38 is connected to the housing window 34 circumferentially between the detent parts 42, 42 of the protective pieces 36, 36 that are adjacent to each other in the circumferential direction. The wall on the distal end side of the housing window 34 is constituted by the detent part 42, and the housing window 34 is located on the proximal end side of the protective piece 36. In addition, the proximal end of the slit 38 is provided at a location off the circumferential center of the housing window 34. Accordingly, the detent parts 42, 42 located on the circumferentially opposite sides of the slit 38 have mutually different protruding dimensions from the columnar part 32 in the circumferential direction.

In the present practical embodiment, the distal end of the slit 38 is provided at a circumferential location corresponding to the circumferential center of the housing window 34. Since a sloping part 44 that slopes in the circumferential direction is provided in the middle of each slit 38, the proximal end of the slit 38 is located off the circumferential center of the housing window 34. That is, the slits 38 is configured such that the lengthwise opposite sides of the sloping part 44 are shifted from each other in the circumferential direction. In addition, each slit 38 has a straight portion that extends in a straight line from the sloping part 44 in the axial direction on each of the distal end side and the proximal end side of the sloping part 44. The sloping part 44 of the slit 38 slopes in the circumferential direction with respect to the axial direction. In the present practical embodiment, the sloping part 44 extends in a straight line at an approximately constant slope angle. The sloping part 44 is provided so as to be smoothly continuous with the axially opposite sides off the sloping part 44 of the slit 38. Therefore, no sharp corners are formed on the wall inner surface of the slit 38.

The slit 38 has a width dimension, which is the distance between the opposed wall inner surfaces, that is larger at the sloping part 44 than at other parts, and the sloping part 44 constitutes the widened part of the present practical embodiment. The width dimension of the slit 38 at the sloping part (the widened part) 44 is desirably set such that when a wing 58 of the winged needle 46 (described later) passes, the wing 58 (a linking part 60) can be housed in the sloping part 44, and is preferably set larger than the axial dimension of the inserted portion (the linking part 60) of the wing 58 inserted into the slit 38. The axial dimension of the sloping part 44 is preferably not greater than half the axial length of the protective piece 36, and more preferably not greater than one-third thereof. The sloping part 44 of the slit 38 is provided at a location away from the housing window 34 to the distal end side, and the portion of the slit 38 located between the sloping part 44 and the housing window 34 is constricted by the detent parts 42, 42 so as to be narrower than the sloping part 44.

The sloping part 44 is preferably located on the proximal end side of the axial center of the protective piece 36. The axial center of the sloping part 44 is desirably located within a range of one-third of the axial dimension of the protective piece 36 from the proximal end of the protective piece 36, and more desirably, within a range of one-fourth of the axial dimension of the protective piece 36 from the proximal end of the protective piece 36.

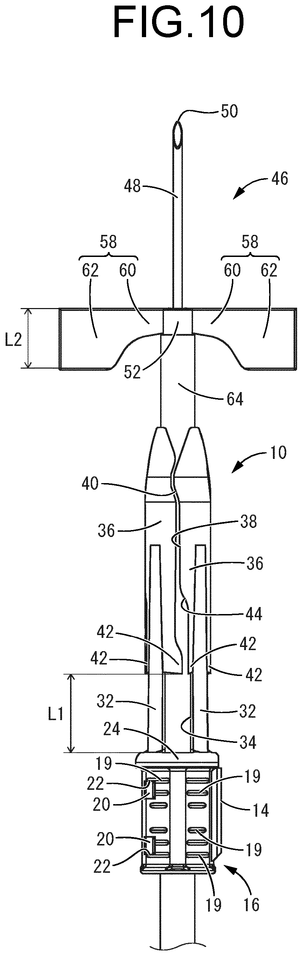

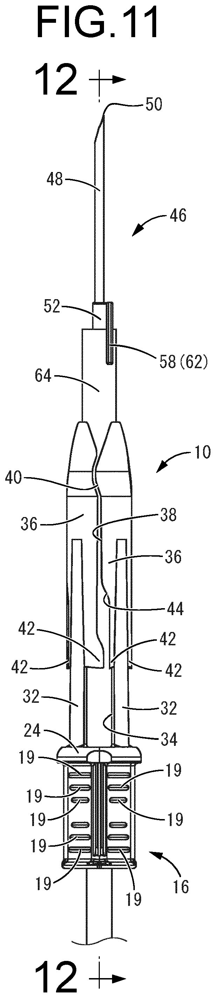

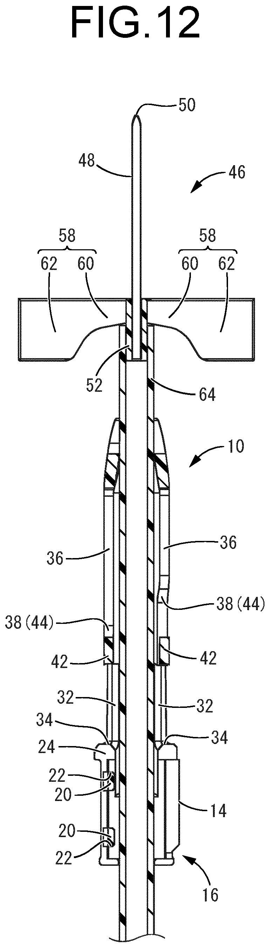

As shown in FIGS. 10 to 12, the needle tip protector 10 is used by being attached to a winged needle 46. The winged needle 46 has a puncture needle 48. The puncture needle 48 is formed of medical-use stainless steel or the like, and includes a needle tip 50 at its distal end. Whereas the puncture needle 48 may be solid, in the present practical embodiment, the puncture needle 48 is a hollow needle.

A needle hub 52 is fixed to the proximal end part of the puncture needle 48. The needle hub 52 has a tubular shape and is fixed to the proximal end part of the puncture needle 48. Besides, two wings 58, 58 protruding from the needle hub 52 to the opposite outer sides are integrally provided with the needle hub 52.

Each wing 58 has a plate shape, and is provided so as to be in contact with the needle hub 52, which has an approximately circular cross section. Accordingly, the wing 58 is provided at a location shifted to one side from the center axis of the needle hub 52 in the radial direction of the needle hub 52 (in the plate thickness direction of the wing 58). The wing 58 includes a linking part 60 that protrudes outward from the needle hub 52, and a wing main body 62 that is provided continuously on the outer side of the linking part 60. The axial length dimension of the wing main body 62 is larger than the axial length dimension of the linking part 60. In the wing 58, a linking part 60 is provided so as to be connected to the distal end portion of the needle hub 52, and the needle hub 52 protrudes toward the proximal end side of the linking part 60. Regarding the wing 58 in the present practical embodiment, the linking part 60 and the wing main body 62 have approximately the same thickness dimension, but the thickness dimensions of the linking part 60 and the wing main body 62 may differ from each other. For example, by making the linking part 60 thinner than the wing main body 62, it is possible to make the wing 58 easier to bend at the linking part 60, and also easier to insert the linking part 60 of the wing 58 into the slit 38.

As shown in FIG. 10, the axial length dimension L1 of the housing window 34 of the needle tip protector 10 is larger than the axial length dimension L2 of the wing main body 62 at the linking part 60 side end. Preferably, the axial length dimension L1 of the housing window 34 is 1.5 times or less as large as the axial length dimension L2 of the wing main body 62 at the linking part 60 side end, which makes it possible to prevent the needle tip protector 10 from becoming excessively large while reliably obtaining ease of insertion of the wing 58 into the housing window 34.

A tube 64 is attached to the needle hub 52. The tube 64 is formed of an elastic material such as a resin elastomer, and is flexible and allowed to change its cross-sectional shape. The distal end portion of the tube 64 is externally attached to the needle hub 52. Preferably, the tube 64 is fixed in place by means such as adhesion and welding.

As shown in FIGS. 10 to 12, the needle tip protector 10 is externally attached to the tube 64. In other words, the tube 64 is inserted through the tubular needle tip protector 10, and the puncture needle 48 and needle hub 52 are located on the distal end side beyond the needle tip protector 10. The tube 64 is inserted through the tube insertion part 16 of the needle tip protector 10, and is positioned and held in contact with the inner circumferential surface of the tube insertion part 16, which is constituted by the tube retaining surfaces 28, 28, 28, 28, and the inner circumferential surface of the proximal end opening 18 of the tube insertion part 16. With the needle tip protector 10 attached to the tube 64, the tube retaining grooves 30 open radially inward toward the tube 64.

For example, during infusion or dialysis, the winged needle 46 is used to puncture a blood vessel of a patient with the puncture needle 48. Then, the infusion bag, the dialysis machine, or the like (not shown) connected to the tube 64 are placed in communication with the blood vessel of the patient via the winged needle 46, so as to perform blood collection, blood return (blood transfusion), medication (infusion), and the like, for example. By folding the two wings 58, 58 at the respective linking parts 60 in the direction of wrapping the needle hub 52, it is possible to use the winged needle 46 for puncture while pinching the two wings 58, 58 overlapped with each other. Besides, with the puncture needle 48 stuck in the blood vessel, by fixing the two wings 58, 58 to the skin of the patient using adhesive tape or the like, the winged needle 46 can be indwelled with the puncture needle 48 stuck in the blood vessel.

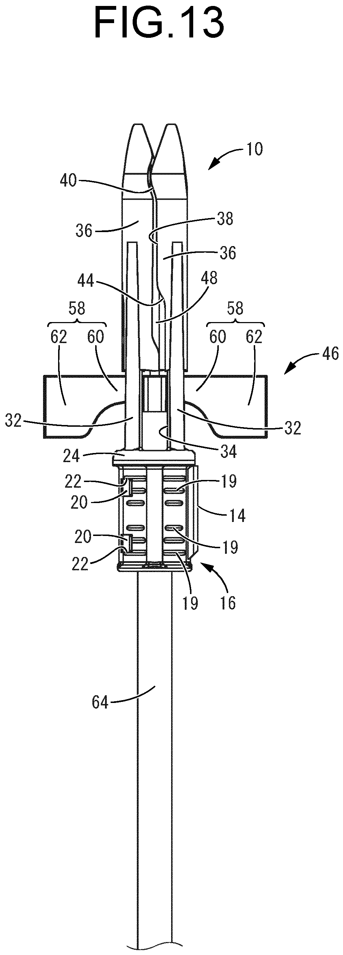

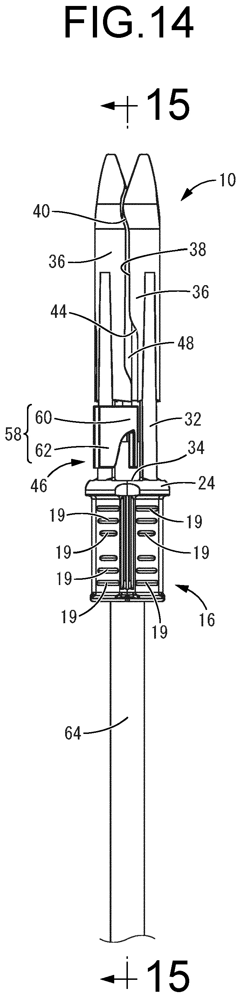

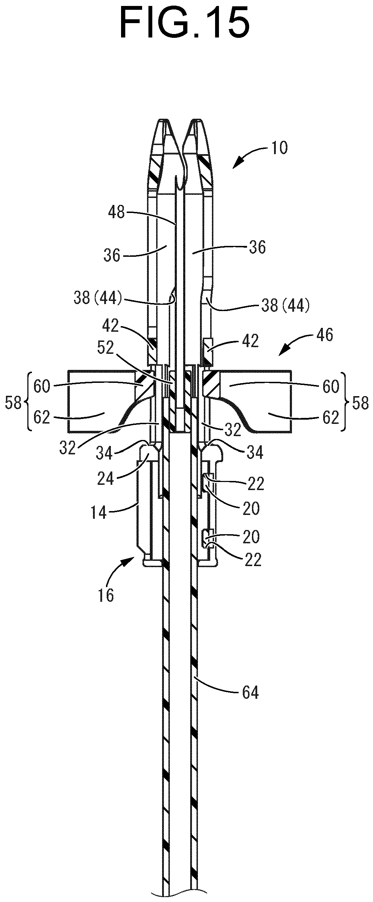

The puncture needle 48 of the winged needle 46 is removed from the blood vessel of the patient after use. When the puncture needle 48 is removed, as shown in FIGS. 13 to 15, the puncture needle 48 is housed in and protected by the needle tip protector 10.

Specifically, the winged needle 46 is removed from the blood vessel by, for example, a physician or the like holding and positioning the needle tip protector 10 in their hand while pulling the tube 64 towards the proximal end side. By pulling the winged needle 46 toward the proximal end side while positioning the needle tip protector 10, the removed winged needle 46 moves towards the proximal end side (near side) relative to the needle tip protector 10, and approaches the needle tip protector 10 from the distal end side. In other words, the needle tip protector 10 moves to the needle tip 50 side relative to the winged needle 46.

The wings 58 of the winged needle 46 approach the needle tip protector 10 in the axial direction from the distal end side, and are inserted into the slits 38 of the needle tip protector 10. Although the wing main bodies 62 may be inserted into the slits 38, in the present practical embodiment, the linking parts 60, whose axial dimension is smaller, are inserted into the slits 38. This reduces the contact area between each wing 58 and the corresponding protective piece 36, thereby decreasing the sliding resistance when the wing 58 moves within the slit 38.

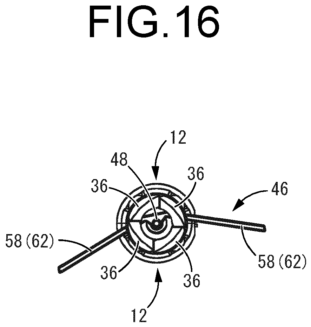

Normally, as shown in FIG. 16, two wings 58, 58 are inserted into the slits 38, 38 on the diametrically opposite sides of the needle tip protector 10. In this case, the two protective pieces 36, 36 are located in one circumferential interval between the two wings 58, 58 inserted into the slits 38, 38, and the other two protective pieces 36, 36 are located in the other circumferential interval between the two wings 58, 58. This insertion mode of the two wings 58, 58 into the slits 38, 38 is referred to as a "2:2 insertion".

Since the distal end portion of the protective piece 36 is tapered, the wing 58 that contacts the distal end portion of the protective piece 36 is guided into the slit 38. The thickness dimension of the wing 58 is larger than the width dimension of the slit 38 at the portions away from the sloping part 44, and the wing 58 moves within the slit 38 toward the proximal end side while pushing the slit 38 open. In addition, the slit 38 is pushed to be wider when the wing 58 passes through the curved part 40 provided at the distal end portion of the slit 38.

As shown in FIGS. 13 to 15, each wing 58 that has passed through the slit 38 is inserted into the corresponding housing window 34. With the wing 58 housed in the housing window 34, the puncture needle 48 is located on the radially inner side of the protective pieces 36, 36, 36, 36, and is surrounded and protected by the protective pieces 36, 36, 36, 36 over approximately the entire circumference. This avoids the situation in which someone accidentally touches the needle tip 50 of the puncture needle 48, and also makes it difficult to see the puncture needle 48 after use from the outside.

With the puncture needle 48 protected by the needle tip protector 10, movement of the winged needle 46 towards the distal end side relative to the needle tip protector 10 is restricted. That is, the slit 38 through which the wing 58 passes is configured such that its distal end opens at a circumferential location approximately corresponding to the circumferential center of the housing window 34, while its proximal end communicates with the housing window 34 at a location off the circumferential center of the housing window 34. With this configuration, when the wing 58 passes through the proximal end of the slit 38, a force in the torsional direction or the like is acting on the winged needle 46, and by the wing 58 entering the housing window 34, the said acting force is released and the wing 58 is located approximately at the circumferential center within the housing window 34. Therefore, even if a force acts to move the winged needle 46 toward the distal end side relative to the needle tip protector 10, the wing 58 is less likely to enter into the slit 38, and the wing 58 comes into contact with the detent part 42, thereby preventing the winged needle 46 from moving toward the distal end side relative to the needle tip protector 10. This makes it possible to prevent the puncture needle 48 from protruding toward the distal end side beyond the needle tip protector 10 again and being exposed, thereby preventing accidental puncture of the puncture needle 48 after use.

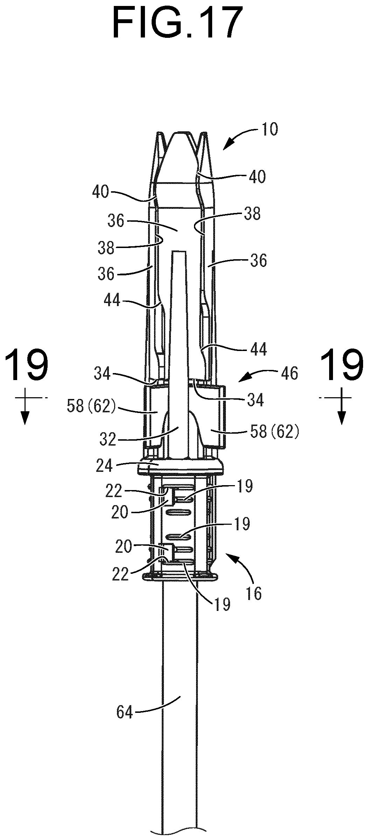

Meanwhile, as shown in FIGS. 17 to 19, there may be a case where the two wings 58, 58 of the winged needle 46 are inserted into the slits 38, 38 located on the opposite sides of one protective piece 36 of the needle tip protector 10. In this case, one protective piece 36 is located in one circumferential interval between the two wings 58, 58 inserted into the slits 38, 38, and the other three protective pieces 36, 36, 36 are located in the other circumferential interval between the two wings 58, 58. This insertion mode of the two wings 58, 58 into the slits 38, 38 is referred to as a "3:1 insertion".

When the two wings 58, 58 are inserted into the slits 38, 38 in such a 3:1 insertion mode, the two wings 58, 58 are in a deformed state in which they are close to each other on one circumferential side. Thus, the elastic recovery force of the two wings 58, 58 acts on each of the protective pieces 36 that constitute the wall of the slits 38, 38.

In addition, by the contact reaction force against the protective piece 36 acting on the wing 58, strain deformation of the wing 58 may be caused, so that a portion of the wing 58, such as the linking part 60 side end of the wing main body 62, which has a longer axial length dimension than that of the needle hub 52 side portion of the wing 58 (the linking part 60), may be inserted into the slit 38. The needle tip protector 10 is configured such that the axial length dimension L1 of the housing window 34 is larger than the axial length dimension of the inserted portion of the wing 58 that is assumed to be inserted into the housing window 34 in the 3:1 insertion mode described above. Thus, even in the case of such a 3:1 insertion mode, the inserted portion of the wing 58 can be stably housed in the housing window 34 without getting caught on the opening peripheral edge of the housing window 34. In the present practical embodiment, the axial length dimension L1 of the housing window 34 is set to be larger than the axial length dimension L2 of the linking part 60 side end of the wing main body 62 or the axial length dimension of the wing main body 62.

The two wings 58, 58 inserted into the housing windows 34, 34 are pressed against the columnar parts 32, 32 and located at the circumferential ends of the housing windows 34, 34. The slit 38 communicates with the corresponding housing window 34 at a location off the circumferentially opposite ends of the housing window 34. Therefore, even if the winged needle 46 attempts to move toward the distal end side relative to the needle tip protector 10, the wing 58, which is located at the circumferential end of the housing window 34, comes into contact with the detent parts 42 of the protective pieces 36 provided on the opposite sides of the slit 38, whereby the wing 58 finds it difficult to enter the slit 38 from the housing window 34. As a result, the movement of the winged needle 46 towards the distal end side relative to the needle tip protector 10 is prevented, and the puncture needle 48 of the winged needle 46 is less likely to protrude toward the distal end side of the needle tip protector 10, thereby preventing accidental puncture due to re-exposure of the needle tip 50 or the like.

In the 3:1 insertion, the wing 58 may be pushed against the columnar part 32 while being housed in the housing window 34. In the present practical embodiment, as shown in FIG. 6, the columnar part 32 has a circumferential angle α about the center axis of the needle tip protector 10 that is not greater than 30 degrees, and the circumferential width dimension of the columnar part 32 is made small. Thus, the circumferential width dimension of the housing window 34 is made large, and the circumferential range of movement of the wing 58 within the housing window 34 is largely obtained. Therefore, compared to the case where the circumferential width dimension of the columnar part is large and the circumferential width dimension of the housing window is small, the contact force of the wing 58 against the columnar part 32 is reduced, and an amount of displacement of the protective piece 36, which is continuous with the columnar part 32, is suppressed. Therefore, the four protective pieces 36, 36, 36, 36 are held in a state of being arranged side by side in a tubular shape, and the puncture needle 48 is prevented from being exposed to the outside through the gaps among the protective pieces 36, 36, 36, 36.

The columnar part 32 has a circumferential angle α that is not smaller than 10 degrees, which reliably obtains the necessary deformation rigidity. This suppresses the displacement of the protective piece 36 due to the deformation of the columnar part 32, thereby stably maintaining the protected state of the puncture needle 48 by the protective pieces 36, 36, 36, 36.

In addition, in the 3:1 insertion, due to the contact reaction force between the wing 58 and the needle tip protector 10 acting on the winged needle 46, the winged needle 46 may incline while being housed in the needle tip protector 10. In that case, it is conceivable that the tube 64 may come into contact with the inner circumferential surface of the annular retaining part 24 of the tube insertion part 16. Regarding the needle tip protector 10 of the present practical embodiment, the inner circumferential surface of the annular retaining part 24 has a quadrangular cross-sectional shape. Thus, the tube 64, which is pressed against the inner circumferential surface of the annular retaining part 24, is easily held in the corners 26 of the annular retaining part 24. The corners 26 of the annular retaining part 24 are arranged at locations that are shifted by approximately 90 degrees in the circumferential direction from the divided location of the tube insertion part 16, which is constituted by combining the division units 12, 12. Therefore, even if the tube 64 held in the corners 26 of the annular retaining part 24 is pressed against the annular retaining part 24, the force due to the contact is unlikely to act directly on the divided location of the division units 12, 12. This prevents troubles such as the division units 12, 12 being pushed open.

Furthermore, in the 3:1 insertion, the tube 64 may be pressed against the tube retaining surfaces 28, which constitute the inner surface of the tube insertion part 16, on the proximal end side of the annular retaining part 24. Since the edge between the two tube retaining surfaces 28, 28 is located at the divided portion of the division units 12, 12, there is a risk that the tube 64, which is guided to the said edge along the tube retaining surface 28, may push open the divided portion of the division units 12, 12. In the present practical embodiment, the tube retaining groove 30 is formed at the end of the tube retaining surface 28 on the distal end side, with which the tube 64 can come into contact. With this configuration, a part of the outer circumferential surface of the tube 64 enters the tube retaining groove 30, so that the tube 64 is held positioned with respect to the needle tip protector 10 in the circumferential direction of the needle tip protector 10. Therefore, the tube 64 is less likely to be guided to the divided portion of the division units 12, 12 due to contact with the tube retaining surface 28, thereby preventing problems such as the division units 12, 12 being pushed open by the tube 64 being pressed against the divided portion of the division units 12, 12.

Each corner 26 of the annular retaining part 24 and the corresponding tube retaining groove 30 of the tube retaining surface 28 are located at approximately the same location in the circumferential direction, and are continuous with each other in the axial direction. Therefore, the positioning action on the tube 64 in the circumferential direction is more effectively exerted, so that the tube 64 is unlikely to be pressed against the divided portion of the division unit 12, 12.

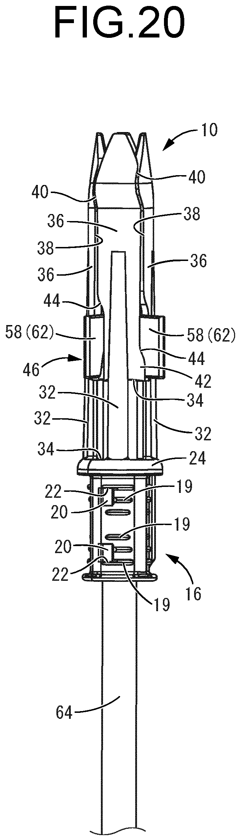

Besides, in the 3:1 insertion, if the slit has a sloping portion or a curved portion, when the wing 58 passes through the said portion, the wing 58 is pressed strongly against the wall inner surface of the slit (the protective piece). This may make it difficult for the wing 58 to pass through the slit, or may significantly deform the protective piece thereby posing a risk of adversely affecting the protective function. In the present practical embodiment, the slit 38 is partially widened to constitute the widened part at the sloping part 44 provided in the middle of the slit 38, and the contact force between the wall inner surface of the slit 38 and the wing 58 is reduced when the wing 58 passes through the sloping part 44. That is, as shown in FIG. 20, the wing 58 passing through the sloping part 44 is housed in the wide sloping part 44, which is able to prevent excessive deformation of the wing 58 due to the sloping of the slit 38 and excessive deformation of the protective piece 36 due to the contact of the wing 58 with the sloping part 44 of the slit 38. Therefore, the sliding resistance during the wing 58 passing through the sloping part 44 of the slit 38 is reduced, thereby making it easier for the wing 58 to pass through the slit 38 having the sloping part 44. Besides, the force acting on the protective pieces 36 during the wing 58 passing through the sloping part 44 of the slit 38 is reduced, thereby holding the four protective pieces 36, 36, 36, 36 in a tubular arrangement.

In addition, the sloping part 44 of the slit 38, which is partially widened, is provided at a location away from the housing window 34 toward the distal end side, and the detent parts 42, 42 provide a constricted portion between the sloping part 44 and the housing window 34, which are both wide. This constricted portion makes it difficult for the wing 58 inserted into the housing window 34 to return to the slit 38. Moreover, the axial length dimension of the said constricted portion is smaller than the axial length dimension L2 of the linking part 60 side end of the wing main body 62 that passes through the slit 38 during the 3:1 insertion. Thus, when the linking part 60 side end of the wing main body 62 passes through the said constricted portion, the wing main body 62 is allowed to incline by at least one of the sloping part 44 and the housing window 34. This suppresses the contact pressure between the wing main body 62 and the protective piece 36 during passing through the said constricted portion, thereby reducing the opening of the protective piece 36 and reducing the resistance when housing the winged needle 46 in the needle tip protector 10.

With the winged needle 46 protected by the needle tip protector 10, the sloping part 44 is located on the proximal end side beyond the needle tip 50 of the puncture needle 48. Accordingly, even if the puncture needle 48 inclines with respect to the needle tip protector 10, the needle tip 50 will not be exposed laterally through the wide sloping part 44, thereby preventing accidental puncture of the needle tip 50.

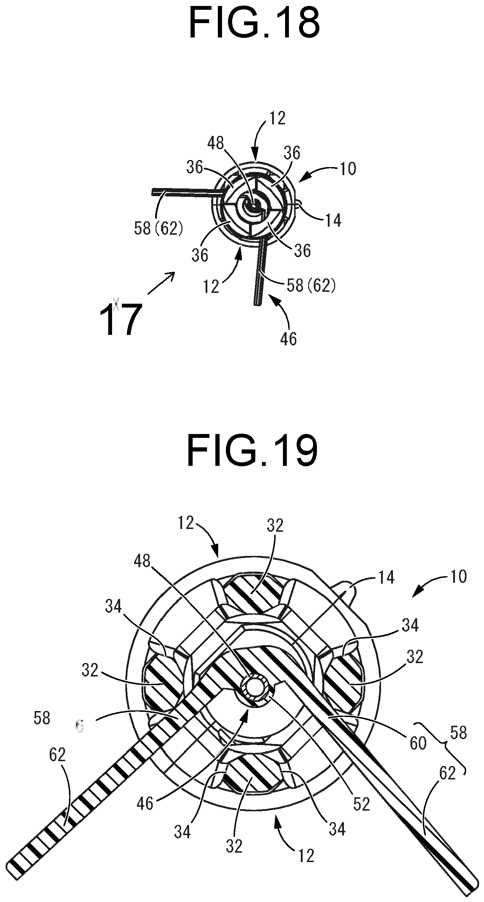

In the preceding practical embodiment, FIGS. 17 to 20 show an example of the 3:1 insertion mode of the wings 58, 58 with respect to the needle tip protector 10. However, regarding the wings 58, 58 inserted in the 3:1 insertion mode, the working effects as described above can be similarly exerted in every case where the wings 58, 58 are inserted into two housing windows 34, 34 that are adjacent to each other in the circumferential direction with any one of the columnar parts 32 interposed therebetween. Furthermore, the wings 58, 58 may be bent to either side in the circumferential direction with respect to the puncture needle 48. For example, in FIG. 19, both the wings 58, 58 are bent downward to the left and right with respect to the puncture needle 48. However, both the wings 58, 58 may be bent upward to the left and right with respect to the puncture needle 48, and even in such a 3:1 insertion mode with respect to the needle tip protector 10, the working effects as described above can be similarly exerted. Besides, in consideration of puncture operation of the puncture needle 48 on the human body, the wing 58 may be provided with a thin-walled part extending in the axial direction in the linking part 60. Even if a portion thinner than the wing main body 62 is provided in the linking part 60 by such a thin-walled part, the effect of the present invention as described above can be effectively exerted.

While the present invention has been described in detail hereinabove in terms of the practical embodiment, the invention is not limited by the specific description thereof. For example, the division units 12, 12 constituting the needle tip protector 10 do not necessarily have to be coupled by hinges 14 at one circumferential end. Specifically, for example, a needle tip protector having a divided structure can be constituted by mutually independent division units 12, 12 being fixed at the circumferentially opposite ends.

Besides, the fixing means for fixing the division units 12, 12 to each other is not limited to a structure in which the coupling hooks 20 each having a claw at its distal end are locked to the peripheral edges of the respective coupling holes 22. Specifically, for example, the fixing means may be constituted by fitting a convex part of one division unit 12 into a concave part of the other division unit 12, or by means such as welding and bonding.

In the preceding practical embodiment, only a single sloping part 44 is provided in the middle of the slit 38, but for example, a plurality of sloping parts may be provided in the middle of the slit 38. When a plurality of sloping parts are provided, it is desirable for the slit 38 to be wide at each sloping part, but it is not necessary for the slit 38 to be wide at all the sloping parts, and for example, there may be wide sloping parts and non-wide sloping parts in a mixed manner. In addition, the plurality of sloping parts may have different slope angles from each other. Besides, the sloping part is not limited to a linear sloping shape whose slope angle is approximately constant, and may be, for example, a curved sloping shape whose slope angle continuously varies, a polygonal-line sloping shape whose slope angle continuously varies, or the like.

The structure of the winged needle 46 can be changed as appropriate. For example, the tube 64 may be fixed in an inserted state to the needle hub 52. In addition, the shape of the wing 58 is not limited, and for example, the entire wing 58 may have approximately the same axial dimension. Furthermore, the wing 58 may have grain-like slip stoppers on the surface to be picked up with the fingers when puncturing, or may have alignment parts to be loosely fitted together on the surfaces that is overlapped with each other when puncturing, or may have markings indicating the thickness of the puncture needle 48.

The preceding practical embodiment exemplifies a needle tip protector 10 including all of the features according to the first preferred embodiment, the third preferred embodiment, the fifth preferred embodiment, the eighth preferred embodiment, the ninth preferred embodiment, the tenth preferred embodiment, and the eleventh preferred embodiment. However, it would also be possible to adopt any one of the first preferred embodiment, the third preferred embodiment, the fifth preferred embodiment, the eighth preferred embodiment, the ninth preferred embodiment, the tenth preferred embodiment, and the eleventh preferred embodiment, or to adopt some of the selected preferred embodiments in combination.

### KEYS TO SYMBOLS

- 10: needle tip protector for a winged needle
- 12: division unit
- 14: hinge
- 16: tube insertion part
- 18: proximal end opening
- 19: anti-slip projection
- 20: coupling hook
- 22: coupling hole
- 24: annular retaining part
- 26: corner
- 28: tube retaining surface
- 30: tube retaining groove
- 32: columnar part
- 34: housing window
- 36: protective piece
- 38: slit
- 40: curved part
- 42: detent part
- 44: sloping part (widened part)
- 46: winged needle
- 48: puncture needle
- 50: needle tip
- 52: needle hub
- 58: wing
- 60: linking part
- 62: wing main body
- 64: tube

## Claims

1. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces; and
four housing windows each provided on a proximal end side of the corresponding protective piece, each housing window being connected to the corresponding slit, wherein
each slit is connected to the corresponding housing window at a location off a circumferential center and circumferentially opposite ends of the housing window.

2. The needle tip protector according to claim 1, wherein a sloping part sloping in a circumferential direction is provided in a middle of each slit, and each slit is configured such that opposite sides of the sloping part are shifted from each other in the circumferential direction.

3. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces;
four housing windows each provided on a proximal end side of the corresponding protective piece, each housing window being connected to the corresponding slit; and
columnar parts separating the housing windows that are adjacent to each other in a circumferential direction, each columnar part being provided circumferentially between adjacent ones of the housing windows while extending from the corresponding protective piece to the proximal end side, wherein
a circumferential angle of each columnar part about a center axis is not greater than 30 degrees.

4. The needle tip protector according to claim 3, wherein the circumferential angle of each columnar part is not smaller than 10 degrees.

5. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces;
four housing windows each provided on a proximal end side of the corresponding protective piece, each housing window being connected to the corresponding slit;
a tube insertion part provided on the proximal end side of the housing windows, the tube insertion part being configured to allow a tube connected to the proximal end side of the winged needle to be inserted; and
an annular retaining part provided at an end of the tube insertion part on a side of the housing windows, the annular retaining part being continuous about an entire circumference, wherein
an inner circumferential surface of the annular retaining part has a quadrangular cross-sectional shape including corners at four locations in a circumferential direction.

6. The needle tip protector according to claim 5, wherein
the tube insertion part has a divided structure divided in the circumferential direction such that the four protective pieces are separated into two pieces each, and
the corners of the inner circumferential surface of the annular retaining part are shifted from a divided location of the tube insertion part in the circumferential direction.

7. The needle tip protector according to claim 5 or 6, wherein
a tube retaining surface configured to retain the tube is provided on an inner surface of the tube insertion part,
a tube retaining groove opening toward the tube is formed at an end of the tube retaining surface on the side of the housing windows, and
the tube retaining groove is located on the proximal end side of each corner of the annular retaining part in the circumferential direction.

8. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces;
four housing windows each provided on a proximal end side of the corresponding protective piece, each housing window being connected to the corresponding slit; and
a tube insertion part provided on the proximal end side of the housing windows, the tube insertion part being configured to allow a tube connected to the proximal end side of the winged needle to be inserted, wherein
a tube retaining surface configured to retain the tube is provided on an inner surface of the tube insertion part, and
a tube retaining groove opening toward the tube is formed at an end of the tube retaining surface on a side of the housing windows.

9. A needle tip protector for a winged needle configured to protect the winged needle having two wings, comprising:
four protective pieces arranged side by side in a tubular shape; and
slits configured to allow the wings of the winged needle to be inserted, each slit being formed circumferentially between adjacent ones of the protective pieces, wherein
a sloping part sloping in a circumferential direction is provided in a middle of each slit, and
each slit is widened at the sloping part.

10. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces; and
four housing windows each provided on a proximal end side of the corresponding protective piece, each housing window being connected to the corresponding slit while being wider than the corresponding slit in a circumferential direction , wherein
each slit includes a widened part whose width dimension is partially increased, the widened part being provided at a location away from the corresponding housing window to a distal end side.

11. A needle tip protector for a winged needle configured to protect the winged needle having two wings, comprising:
four protective pieces arranged side by side in a tubular shape;
slits configured to allow the wings of the winged needle to be inserted, each slit being formed circumferentially between adjacent ones of the protective pieces; and
four housing windows each provided on a proximal end side of the corresponding protective piece, each housing window being connected to the corresponding slit, wherein
in every state where the two wings are inserted into any two of the housing windows that are adjacent to each other in a circumferential direction, an axial length of each housing window is larger than that of an inserted portion of each wing inserted into the corresponding housing window such that the inserted portion of each wing is housed within the corresponding housing window.
